# EUROPEAN PATENT SPECIFICATION

(11) **EP 3 060 923 B1**
(45) Date of publication and mention of the grant of the patent: **28.02.2018**
(21) Application number: 14802222.1
(22) Date of filing: 25.10.2014
(51) Int. Cl.: G01N 33/68

(54) **A METHOD OF DETECTING AN INITIAL PSYCHOSIS EPISODE, PARTICULARLY ONE OF SCHIZOPHRENIC CHARACTER**
VERFAHREN ZUR ERKENNUNG DER ANFANGSEPISODE EINER PSYCHOSE, INSBESONDERE VON SCHIZOPHRENEM CHARAKTER
PROCÉDÉ DE DÉTECTION D'UN ÉPISODE INITIAL DE PSYCHOSE, EN PARTICULIER UN DE CARACTÈRE SCHIZOPHRÈNE

(30) Priority: 25.10.2013 PL 40577213
(43) Date of publication of application: 31.08.2016
(73) Proprietor: Pomorski Uniwersytet Medyczny w Szczecinie, 70-204 Szczecin (PL)
(72) Inventor: KUCHARSKA MAZUR, Jolanta, PL-72-003 Woleczkowo (PL); SAMOCHOWIEC, Jerzy, PL-71-315 Szczecin (PL); RATAJCZAK, Mariusz, PL-32-020 Wieliczka (PL); TARNOWSKI, Maciej, PL-71-220 Bezrzecze (PL); DOLEGOWSKA, Barbara, PL-70-785 Szczecin (PL)
(74) Representative: Witek, Rafal
(86) International application number: PCT/PL2014/050068
(87) International publication number: WO 2015/060739

(56) References cited:
- US-A1- 2002 127 712
- JOLANTA KUCHARSKA-MAZUR ET AL: "Novel evidence for enhanced stem cell trafficking in antipsychotic-naïve subjects during their first psychotic episode", JOURNAL OF PSYCHIATRIC RESEARCH, vol. 49, 1 February 2014 (2014-02-01), pages 18-24, XP055163157, ISSN: 0022-3956, DOI: 10.1016/j.jpsychires.2013.10.016
- ANNA BOYAJYAN ET AL: "Alternative Complement Pathway in Schizophrenia", NEUROCHEMICAL RESEARCH, KLUWER ACADEMIC PUBLISHERS-PLENUM PUBLISHERS, NE, vol. 35, no. 6, 26 January 2010 (2010-01-26), pages 894-898, XP019827333, ISSN: 1573-6903

## Description

The subject of the present invention is a method of detecting an initial psychosis episode by evaluating the levels of serum a marker of. The present invention relates to a novel diagnostic method capable of being used in psychiatry in the detection of changes in peripheral blood accompanying an initial psychosis episode, which make it possible to detect a psychological disturbance and to determine its characteristics.

Psychosis (From the Greek, *psyche*-soul and *osis*-madness) is a psychological abnormality defined in psychiatry as a disease during which at least one of the following symptoms occurs: hallucinations, delusions, or behavioural or linguistic disruptions, that lead to a significant disruption of the assessment of reality, regardless of the cause of these symptoms. Persons in a state of psychosis are often convinced of the reality of their experience and perceive that they are functioning normally.

Psychoses are treated pharmacologically. Outcome predictions are dependent on the type of psychosis. For this reason, therapeutic success particularly depends on the correct diagnosis of a psychosis as well an accurate determination of the character of an initial psychosis episode determined in a patient.

Despite intensive research in this direction, so far no effective and straightforward psychosis biomarkers have been known.

The goal of the present invention is to deliver an objective laboratory test, which could accurately identify an initial psychosis episode, and in particular identify an initial episode of psychosis of schizophrenic character. A particular goal of the present invention is to deliver serum a marker of characteristic of an initial psychosis episode.

Unexpectedly, the above defined goal has been attained by the present invention.

The subject of present invention is the use of the serum level of C3a, a part of the complement system, as well as the serum level of sphingosine-1-phosphate, as a marker of an initial psychotic episode.

The next subject of the present invention is the use of sphingosine-1-phosphate and Lin-/CD45-/CD34+ VSEL stem cell numbers as a marker of an initial psychotic episode, one of schizophrenic character.

The next subject of the present invention is a method of detecting an initial psychosis episode, particularly one of schizophrenic character, characterised in that a patient's collected blood sample is used to determined the level of C3a protein content, the content level of sphingosine-1-phosphate as well as the content level of Lin-/CD45-/CD34+ VSEL stem cells, wherein a serum C3a protein level below about 550 [ng/ml] and a serum level of sphingosine-1-phosphate below about 2.33 [µg/ml] are evidence of the occurrence of an initial psychotic episode, whereas a serum level of sphingosine-1-phosphate below about 2.33 [µg/ml] and Lin-/CD45-/CD34+ VSEL stem cell numbers above about 0,45 [cells/µl of peripheral blood] are evidence of the occurrence of an initial psychotic episode of schizophrenic character.

According to the present invention, it was unexpectedly shown that a serum level of C3a below about 550 [ng/ml] and the serum level of sphingosine-1-phosphate below about 2.33 [µg/ml] are a marker of an initial psychotic episode, whereas the serum level of sphingosine-1-phosphate below about 2.33 [µg/ml] and Lin-/CD45-/CD34+ VSEL stem cell numbers above about 0.45 [cells/µl of peripheral blood] are a marker of an initial psychotic episode of schizophrenic character.

Extant prior art does not describe the role of VSELs nor of the factors responsible for their migration in the aetiology of an initial psychotic episode.

In the view of the authors of the present invention, the role of factors connected with regenerative processes as a potential link with the immunological, neurodegenerative and neuro-ontological theories of schizophrenia, as well as the glutamate theory of psychosis development. The results obtained in accordance with the present invention, are on one hand evidence of the existence of a common mechanism of initiating an initial psychotic episode, as evidence by the role of sphingosine-1 phosphate therein, regardless of aetiology, but also point out to numerous differences between psychoses depending on a particular diagnosis: schizophrenic or non-schizophrenic psychosis. The results obtained unexpectedly show that intensive regenerative processes occur in persons with an initial episode of psychosis (in which stem cells take part directly or through paracrine interactions). Thus far, during an initial episode of psychosis, no systematic analysis of two substances responsible for stem cell migration has been made (Ratajczak et al. 2010; Ratajczak et al. 2012): the C3a protein, an element of the complement cascade, which participates in brain cell response to injury factors (Bitzer-Quintero and González-Burgos 2012; Lettiero et al. 2012), as well as sphingosine-1-phosphate, a lysophospholipid involved in neurogenesis (Harada et al. 2004; Kimura et al. 2007).

The research which resulted in the present invention was performed as part of the grant entitled "Innovative methods of using stem cells in medicine" POIG.01.01.02-00-109/09 at the Pomeranian Medical University in Szczecin during the years 2010-2012.

To better illustrate the nature of the present invention defined above, its description has been supplemented with the attached figures as well as the following example embodiments, which should not, however, be seen as limiting its scope.
Figure 1 represents a discriminatory function analysis for both experimental groups relating to the identification of a marker of an initial psychosis for an entire group of psychoses (regardless of the diagnosed type of psychosis) according to the present invention.
Figure 2 represents a discriminating function analysis for both evaluated groups of a marker of an initial psychosis of schizophrenic character according to the present invention.

The present invention will be better explained by the following example embodiments.

### Example 1. Identification of a marker of an initial psychosis episode

### Marking methodology

Morphology was evaluated using an ABX Micros 60 haematological analyser (Horiba). Blood collected onto K₃EDTA was centrifuged (250g; 10 minutes; 20°C). The resulting serum was transferred to a fresh tube and frozen at a temperature of -80°C for further research.

### Determination of sphingosine-1-phosphate using RP HPLC (high-performance liquid chromatography with a reversed phase system)

The serum was defrosted at room temperature, and then supplemented with D-erythro-sphingosine-1-phosphate (S1P C17-internal marker), 1M NaCl and methanol. This was mixed, supplemented with chloroform, mixed again and centrifuged. The lowermost, organic phase was transferred to a fresh tube. the organic phase was supplemented with chloroform, was mixed and was centrifuged. Both organic phases were combined, and dried in a vacuum centrifuge.

The dried residue was dissolved in methanol. We added the reaction mixture (o-phthalyladialdehyde, methanol, mercaptoethanol and boric acid, pH 10.5), incubated and centrifuged. The clarified supernatant was transferred to a fresh tube and analysed using a Hewlett Packard Series 1200 chromatograph. Chromatographic data was evaluated using HP Chemstation software (Hewlett Packard, presently Agilent).

The separation in a reversed phase system (RP-HPLC) was performed in a Cosmosil 5 µm C18-ARII column (150 x 4,6) with a 5 µm C18-ARII pre-column (10 x 4,6) (Waters). The column temperature was 25 °C. We used an isocratic method with a mobile phase consisting of 10 mM K₂HPO₄ (pH 5.5) and methanol (15:85; v/v). The flow rate was 1 ml/min. 50 µl samples were injected every 28 minutes. The S1P derivative detection wavelengths were 340 nm for excitation and 455 nm for the emission. The basis for S1P content calculations was the surface area of the peak in relation to the peak surface area of the internal marker. To identify peaks and for calibration, we used a mixture of the markers S1P C17 and S1P C18 (Avanti Polar Lipids) (Blogowski et al., 2013b; Caligan et al., 2000; Egom et al., 2013; Starzynska et al., 2013).

### Determination of C3a using the Human C3a ELISA Kit (BD OptEIA)

Wells were loaded with standards and diluted sera. The plate was incubated and rinsed then supplemented with a substrate (TMB), incubated again, supplemented with Stop Solution and then absorbance was measured (wavelength of 450 nm) using an EnVision (Perkin-Elmer) plate reader (Blogowski et al., 2012; Blogowski et al., 2013a; Blogowski et al., 2013b; Starzynska et al., 2013).

### Blood analysis using flow cytometry.

Samples of peripheral blood were lysed twice using BD Pharm Lyse lysing buffer (BD Bioscience) at room temperature for 10 minutes and then rinsed with phosphate-buffered physiological saline (PBS) with an addition of 2% foetal bovine serum (FBS; Sigma) to obtain a total pool of nucleated cells (TNCs). Nucleated cells were then stained for markers of hematopoietic cell lines using antibodies conjugated with fluorescein isothiocyanate (FITC) against human: CD2 (clone RPA-2.10); CD3 (clone UCHT1); CD14 (clone M5E2); CD16 (clone 3G8); CD19 (clone HIB19); CD24 (clone ML5); CD56 (clone NCAM16.2); CD66b (clone G10F5); and CD235a (clone GA-R2) (all from BD Bioscience). The cells were simultaneously stained for a panleukocytic marker, CD45, with antibodies conjugated with phycoerythrin (PE) (clone HI30; BD Biosciences) and for one of the following antigens: CD 34 with antibodies conjugated with allophycocyanin (APC) (clone 581; BD Bioscience) or CD 133 (CD133/1, antibodies conjugated with APC; Miltenyi Biotec). Additionally, we used the following isotype controls: antibodies conjugated with FITC-mouse IgG1, κ (clone MOPC-21), mouse IgG2a, κ (clone G155-178), mouse IgG2b, κ (clone 27-35); conjugated with PE-mouse IgG1, κ (clone MOPC-21) and conjugated with APC-mouse IgG1, κ (clone MOPC-21) (all from BD Bioscience). As isotype controls we also used APC-conjugated mouse IgG1 (clone IS5-21F5; Miltenyi Biotec). Staining was performed in PBS with 2 % FBS, on ice, for 30 minutes. Cells were then rinsed, resuspended in liquid and analysed using a NAVIOS flow cytometer (Beckman Coulter). Each sample resulted in at least 106 events. The total number of VSELs and HSCs was calculated (individually per patient) recalculating for 1 ml of peripheral blood based on the percent content of these cells observed cytometrically and the total number of white cells in 1 ml of peripheral blood. The analysis was performed using Kaluza software (Beckman Coulter) (Paczkowska et al., 2009; Zuba-Surma & Ratajczak 2010).

### Study group

Patients for the study group were recruited in the West-Pomeranian District of Poland. All were hospitalised in the SPSK-1 Psychiatry Clinic of the Pomeranian Medical University in Szczecin. The patients were given written information about the study and prior to signing the consent form, they were given answers to all of their questions regarding the study.

The study involved 30 unrelated individuals with a diagnosed initial psychotic episode (understood as a so-far untreated psychosis, during which there was no autologous improvement and regardless of its duration). The nosological diagnosis was set out according to the ICD-10 research diagnostic criteria:
- paranoid schizophrenia (F 20.0)- 11 persons (36,7 %)
- simple schizophrenia (F 20.6)-1 person (3,33%)
- persistent delusional disturbances (F 22)-1 person (3,33%)
- acute polymorphous psychotic disturbances without schizophrenic symptoms (F 23.0)-5 persons (16,7%)
- acute polymorphous psychotic disturbances with schizophrenic symptoms (F 23.1)-6 persons (20 %)
- acute polymorphous psychotic disturbances similar to schizophrenia (F 23.2)-4 persons (13,3%)
- mania with psychotic symptoms (F 30.2)-1 person (3,33%)
- deep depression episode with psychotic symptoms (F 32.2)-1 person (3,33%)

During subsequent observations, following the end of the study and a remission of the psychotic symptoms, the latter two cases were diagnosed with affective bipolar disorders (F31), and for this reason both subjects were treated in the calculations as an initial episode of the bipolar disorders.

The study group was divided into two subgroups:
- "schizophrenic", into which we qualified all persons with the F 20, F23.1 and F 23.2 diagnoses
- "non-schizophrenic" which contained all persons with remaining diagnoses.

### Excluding criteria were:

- lack of conscious, written consent for participating in the study
- the occurrence of organic psychological disorders or other IICD-10 disorders beyond the above-indicated diagnoses
- a connection between present psychological disorders and psychoactive substance use, including alcohol
- serious somatic diseases, in particular those requiring drug treatment
- glucose intolerance
- current, active inflammatory disease (patients were excluded based on laboratory experiments as well as pre-study evaluations)
- use, for any reason, of psychotropic drugs in the present episode and less than half a year prior to the onset of the episode.

Persons from the study group were evaluated by a specialist psychiatrist. An interview was held and a standard psychiatric evaluation was performed as well as a physical and neurological evaluation; accessible family members of the patient were also interviewed. The occurrence of psychological disturbances other than an initial episode of psychosis was excluded using the MINI protocol (Sheehan et al.,1998). The patients were also evaluated with a positive and negative PANSS syndrome scale (Rzewuska 2002)

Demographic data, family interviews as well as the history of symptom occurrence were collected via a standardized disease history.

### Control group

The control group consisted of 35 persons, selected by age, sex, origins, sociodemographic indicators and body mass to match the study group, lacking I ICD-10 axis diagnoses at present and in their interview, and somatically healthy.

These persons were evaluated by a specialist psychiatrist analogously to members of the study group. Demographic data, family interviews as well as disease symptom occurrence histories were collected using a standardised disease history.

### Study procedures

Venous blood was collected from persons in the study group twice, an initial time in the morning, prior to the administration of any antipsychotic drugs, and a second time after the onset of a clinical improvement following treatment with a neuroleptic, understood as a remission of hallucinations and delusions, as well as a score reduction on the PANSS scale by at least 20 %. In relation to "schizophrenic" patients, none of the PANSS scale points indicated in the schizophrenia remission characteristics (P1-delusions, P2-cognitive disorganisation, P3- hallucinatory behaviours, G5-mannerisms and freezing, N1-affect flattening, N4-passivity/apathy, N6-lack of spontaneity and fluency in speech) could not exceed 3.

Blood was immediately transferred for further analyses to the Laboratory of the Faculty and Department of Physiology WMU. On the day of blood collection we also performed a psychiatric evaluation, a physical as well as psychometric scaling and basic laboratory tests, and neuroleptics were administered after blood collection:
- amisulpride, max. 800 mg/day, 24 patients
- olanzapine, max. 20 mg/day, 2 patients
- perazine max. 600 mg/day, 2 patients
- haloperidol, max. 8/day, 1 patient
- quetiapine, max. 600 mg/day, 1 patient.

The dosage was determined based on a patient's tolerance for the drug.

### Results

**Table 1. A comparison of the stem cell numbers between the study and control groups.**

| VSEL [number of cells/µl] (average ± SD) | PG-BT (n=30) | PG-AT (n=30) | SG-BT (n=22) | SG-AT (n=22) | CG (n=35) | P |
|---|---|---|---|---|---|---|
| Lin-/CD45-/CD34+ | 0,3208 ±0,1985 | 0,2718± 0,1780 | 0,3326± 0,1879 | 0,2946± 0,2309 | 0,1705± 0,1031 | * |

| | | | | | | |
|---|---|---|---|---|---|---|
| Legend: PG-study group, SG- "schizophrenic" group, CG-control group, BT-prior to treatment, AT-following treatment We determined the following statistically significant differences: * PG-BT vs. CG (p=0,0006), PG-AT vs. CG (p=0,001), PG-BT vs. PG-AT (p=0,36), SG-BT vs. CG (p=0, 0005), SG-AT vs. CG (p=0,02), NG-AT vs. CG (p=0,003) | | | | | | |

**Table 2. Comparison of the serum level of C3a and sphingosine-1-phosphate in the psychotic and control groups.**

| | PG-BT (n=30) | PG-AT (n=30) | SG-BT (n=22) | SG-AT (n=22) | CG (n=35) | P |
|---|---|---|---|---|---|---|
| C3a [ng/ml] | 499,96 ± 171,99 | 561,49 ± 125,20 | 494,33 ± 169,34 | 562,26 ± 117,73 | 591,13 ± 149,61 | * |
| S1P [µg/ml] | 2,32 ± 0,17 | 2,31± 0,23 | 2,30 ± 0,17 | 2,27 ± 0,26 | 2,45± 0,18 | ** |

| | | | | | | |
|---|---|---|---|---|---|---|
| Legend: PG-study group, SG- schizophrenic group, CG-control group, BT-prior to treatment, AT-following treatment We determined the following statistically significant differences: *PG-BT vs.CG (p =0,036), ale PG-AT vs. CG (p>0,1), SG vs. NG (p>0,1) **PG-BT vs.CG (p=0,0017), PG-AT vs.CG (p=0,0067), SG-BT vs. NG-BT (p=0,007) | | | | | | |

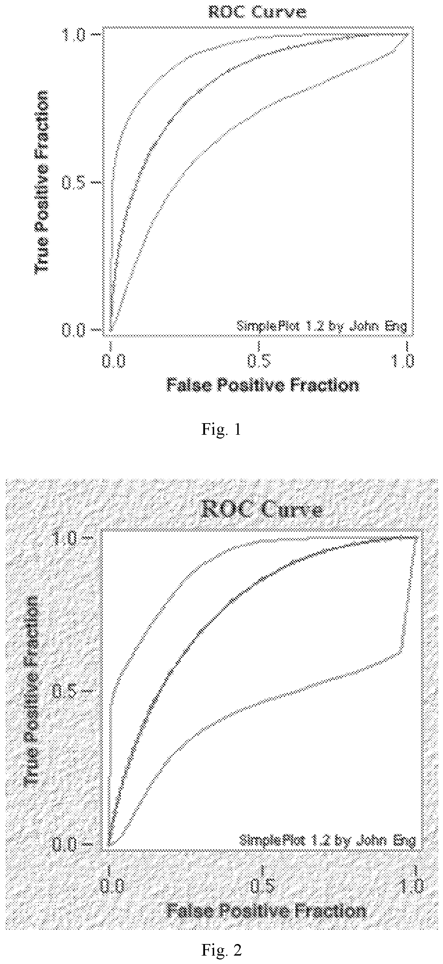

In the following figures we represent discrimination function analyses for both evaluated groups:
1) a marker of an initial psychosis for the whole psychotic group (regardless of diagnosis): for C3a<550 [ng/ml] and S1P<2.33 [µg/ml], p=0,00004 OR=9,58. This model was confirmed using a logistic regression, sub-curve area was ROC = 0.8378 (Fig. 1).
2) a marker of an initial psychosis episode for the schizophrenic group: Number of Lin-/CD45-/CD34+ VSELs > 0,45 [cells/µl], S1P< 2.33 [µg/ml], p = 0,012, OR= 1,5.

This model was confirmed using a logistic regression, sub-curve area was ROC = 0.77 (Fig. 2).

### Bibliography:

Bitzer-Quintero OK, González-Burgos I. Immune system in the brain: a modulatory role on dendritic spine morphophysiology? Neural Plast 2012;2012:348642.
Blogowski W, Budkowska M, Salata D, Serwin K, Dolegowska B, Lokaj M, Prowans P, Starzynska T. Clinical analysis of selected complement-derived molecules in human adipose tissue. Journal of Translational Medicine 2013 a; 11:11.
Blogowski W, Dolegowska B, Budkowska M, Salata D, Domanski L, Starzynska T. Perioperative release of pro-regenerative biochemical signals from human renal allografts subjected to ischemia-reperfusion injury. Innate Immunity 2013 b Apr 22. [Epub ahead of print]
Blogowski W, Dolegowska B, Salata D, Budkowska M, Domański L, Starzyńska T. Clinical analysis of perioperative complement activity during ischemia/reperfusion injury following renal transplantation. Clinical Journal of American Society of Nephrology 2012;7(11):1843-51.
Caligan TB, Peters K, Ou J, Wang E, Saba J, Merrill AH Jr. A high-performance liquid chromatographic method to measure sphingosine 1-phosphate and related compounds from sphingosine kinase assays and other biological samples. Analytical Biochemistry 2000;281(1):36-44.
Egom EE, Mamas MA, Chacko S, Stringer SE, Charlton-Menys V, El-Omar M, Chirico D, Clarke B, Neyses L, Cruickshank JK, Lei M, Fath-Ordoubadi F. Serum sphingolipids level as a novel potential a marker of for early detection of human myocardial ischaemic injury. Frontiers in Physiology 2013;4:130.
Harada J, Foley M, Moskowitz MA, Waeber C. Sphingosine-1-phosphate induces proliferation and morphological changes of neural progenitor cells. J Neurochem 2004;88(4):1026-39.
Kimura A, Ohmori T, Ohkawa R, Madoiwa S, Mimuro J, Murakami T, Kobayashi E, Hoshino Y, Yatomi Y, Sakata Y. Essential roles of sphingosine 1-phosphate/S1P1 receptor axis in the migration of neural stem cells toward a site of spinal cord injury. Stem Cells. 2007;25(1):115-24.
Klasyfikacja zaburzeń psychicznych and zaburzeń zachowania w ICD-10, Badawcze kryteria diagnostyczne, Uniwersyteckie Wydawnictwo Medyczne Vesalius, Instytut Psychiatrii and Neurologii, Kraków-Warszawa, 1998.
Lettiero B, Andersen AJ, Hunter AC, Moghimi SM. Complement system and the brain: Selected pathologies and avenues toward engineering of neurological nanomedicines. J Control Release 2012;161(2):283-9.
Paczkowska E, Kucia M, Koziarska D, Halasa M, Safranow K, Masiuk M, Karbicka A, Nowik M, Nowacki P, Ratajczak MZ, Machalinski B. Clinical evidence that very small embryonic-like stem cells are mobilized into peripheral blood in patients after stroke. Stroke 2009;40:1237-44.
Ratajczak MZ, Lee H, Wysoczynski M, Wan W, Marlicz W, Laughlin MJ, Kucia M, Janowska-Wieczorek A, Ratajczak J. Novel insight into stem cell mobilization-plasma sphingosine-1-phosphate is a major chemoattractant that directs the egress of hematopoietic stem progenitor cells from the bone marrow and its level in peripheral blood increases during mobilization due to activation of complement cascade/membrane attack complex. Leukemia. 2010;24(5):976-85.
Ratajczak MZ, Borkowska S, Ratajczak J. An emerging link in stem cell mobilization between activation of the complement cascade and the chemotactic gradient of sphingosine-1-phosphate. Prostaglandins Other Lipid Mediat. 2013;104-105:122-9
Rzewuska M. Validity and reliability of the Polish version of the Positive and Negative Syndrome Scale (PANSS). International Journal of Methods in Psychiatric Research 2002;11:27-32.
Sheehan DV, Lecrubier Y, Sheehan KH, Amorim P, Janavs J, Weiller E, Hergueta T, Baker R, Dunbar GC. The Mini-International Neuropsychiatric Interview (M.I.N.I.): the development and validation of a structured diagnostic psychiatry interview for DSM-IV and ICD-10. The Journal of Clinical Psychiatry 1998;59 Suppl 20:22-33;quiz 34-57.
Starzynska T, Dabkowski K, Blogowski W, Zuba-Surma E, Budkowska M, Salata D, Dolegowska B, Marlicz W, Lubikowski J, Ratajczak MZ. An intensified systemic trafficking of bone marrow-derived stem/progenitor cells in patients with pancreatic cancer. Journal of Cellular and Molecular Medicine 2013;17(6):792-9.
Zuba-Surma EK, Ratajczak MZ. Overview of very small embryonic-like stem cells (VSEL) and methodology of their identification and isolation by flow cytometric methods. Current protocols in cytometry 2010; Chapter 9: Unit9, 29.

## Claims

1. The use of the serum level of C3a, a component of the complement cascade as well as the serum level of sphingosine-1-phosphate, as a marker of an initial psychotic episode.

2. The use of sphingosine-1-phosphate and the number of Lin-/CD45-/CD34+ VSEL stem cells as a marker of an initial psychotic episode of schizophrenic character.

3. A method of detecting an initial psychosis episode, particularly one of schizophrenic character, **characterised in that** a the level of C3a protein content, the content level of sphingosine-1-phosphate and possibly the content level of Lin-/CD45-/CD34+ VSEL stem cells are determined in a blood sample from a patient, wherein a serum level of the C3a protein below 550 [ng/ml] and a serum level of sphingosine-1-phosphate below 2.33 [µg/ml] are evidence of the occurrence of an initial psychotic episode, whereas particularly the serum level of sphingosine-1-phosphate below 2.33 [µg/ml] and content level of Lin-/CD45-/CD34+ VSEL stem cells above 0,45 [cells/µl of peripheral blood] are evidence of the occurrence of an initial psychotic episode of schizophrenic character.

## Patentansprüche

1. Verwendung des Serumspiegels von C3a, einer Komponente der Komplementkaskade, sowie des Serumspiegels von Sphingosin-1-phosphat, als ein Marker einer initialen psychotischen Episode.

2. Verwendung von Sphingosin-1-phosphat und der Zahl an Lin-/CD45-/CD34+ VSEL Stammzellen als ein Marker einer initialen psychotischen Episode von schizophrenem Charakter.

3. Verfahren zum Nachweis einer initialen psychotischen Episode, insbesondere einer von schizophrenem Charakter, **dadurch gekennzeichnet, dass** der Spiegel von C3a Proteingehalt, der Spiegel an Gehalt von Sphingosin-1-phosphat, und möglicherweise der Spiegel an Gehalt von Lin-/CD45-/CD34+ VSEL Stammzellen in einer Blutprobe von einem Patienten bestimmt werden, wobei ein Serumspiegel des C3a Proteins von unterhalb von 550 [ng/ml] und ein Serumspiegel von Sphingosin-1-phosphat von unterhalb von 2,33 [µg/ml] ein Hinweis auf das Auftreten einer initialen psychotischen Episode sind, wohingegen insbesondere der Serumspiegel von Sphingosin-1-phosphat von unterhalb von 2,33 [µg/ml] und der Spiegel an Gehalt von Lin-/CD45-/CD34+ VSEL Stammzellen von oberhalb von 0,45 [Zellen/µl an peripherem Blut] ein Hinweis auf das Auftreten einer initialen psychotischen Episode von schizophrenem Charakter sind.

## Revendications

1. Utilisation du taux sérique de C3a, un composant de la cascade du complément ainsi que le taux sérique de sphingosine-1-phosphate, en tant que marqueur d'un épisode initial de psychose.

2. Utilisation de sphingosine-1-phosphate et du nombre de cellules souches VSEL Lin-/CD45-/CD34+ en tant que marqueur d'un épisode initial de psychose de caractère schizophrène.

3. Méthode de détection d'un épisode initial de psychose, particulièrement un de caractère schizophrène, **caractérisée en ce que** le taux de teneur en protéine C3a, le taux de teneur en sphingosine-1-phosphate et éventuellement le taux de teneur en cellules souches VSEL Lin-/CD45-/CD34+ sont déterminés dans un échantillon de sang provenant d'un patient, dans laquelle un taux sérique de la protéine C3a inférieur à 550 [ng/ml] et un taux sérique de sphingosine-1-phosphate inférieur à 2,33 [µg/ml] sont la preuve de l'occurrence d'un épisode initial de psychose, tandis que, en particulier, le taux sérique de sphingosine-1-phosphate inférieur à 2,33 [µg/ml] et le taux de teneur en cellules souches VSEL Lin-/CD45-/CD34+ supérieur à 0,45 [cellules/µl de sang périphérique] sont la preuve de l'occurrence d'un épisode initial de psychose de caractère schizophrène.
